# EUROPEAN PATENT SPECIFICATION

(11) **EP 2 251 133 B1**
(45) Date of publication and mention of the grant of the patent: **02.07.2014**
(21) Application number: 09160436.3
(22) Date of filing: 15.05.2009
(51) Int. Cl.: B23K 26/06, B23K 26/00, A61F 2/00

(54) **Method for generating a surface structure**
Verfahren zur Erzeugung einer Oberflächenstruktur
Procédé de génération d'une structure de surface

(43) Date of publication of application: 17.11.2010
(73) Proprietor: Swiss Micro Laser GmbH, 8143 Stallikon (CH)
(72) Inventor: Bak, Marco, 2691 AK, 's-Gravenzande (NL); Van Merksteijn, Jacobus Lambertus, 3920 Zermatt (CH)
(74) Representative: de Lange, Hendrik Cornelis

(56) References cited:
- WO-A1-2005/084586
- WO-A1-2007/091155
- DE-A1- 10 157 316
- US-A- 5 961 552
- US-A1- 2005 211 680
- US-A1- 2008 299 408

## Description

The invention relates to a method of generating surface structures. These surface structures are for instance used in bone implants, where titanium or titanium alloy elements are partly or completely inserted in a bone. In order to strengthen the bonding between bone tissue and the implant and in order to promote the growth of the bone tissue on and into the surface structure, the surface structure of the implant is roughened. One way of roughening the surface of such implants is by applying particle impingement such as shot blasting. Another roughening technique that is applied is etching of the surface.

For instance in dental inserts in order to generate a suitable roughness the surface structure of the inserts are roughened in two steps. First a rough surface structure is obtained by particle impingement where after a much finer roughening is superposed on the rough surface structure by etching. These techniques however have some serious drawbacks. For instance while impinging the material with particles some of the particle material will stay behind on the surface, providing unwanted contamination. Such contamination in bone implants is evidently highly undesirable.

A drawback of the etching technique is that the etching process is very difficult to control and, furthermore the etching induces aggressive etching substances eating away and reacting with surface. These substances can similarly introduce contamination of the surface structure.

As alternative it has been proposed (see US-A-2005 0211680) to apply laser induced surface etching instead. Although this technique has proven to be superior with respect to the contamination, the roughness of the surface structure still leaves room for improvement.

The object of the invention is to provide an alternative method of generating a surface structure, which alleviates the drawbacks of the art while maintaining and/or improving the advantages thereof. This object is reached by a method for producing a surface structure wherein a substrate is provided having a surface to be treated wherein a portion of the surface is irradiated with a beam of a pulsed ultra violet laser projecting a spot on the surface, wherein the pulsed UV laser is having a pulse length of 5 to 10 picoseconds and a pulse frequency of 200 to 300 kiloherz, wherein the energy density per pulse ranges from 0.003 to 0.5 microjoules per square micrometer, wherein the spot is moved over the surface to be treated and wherein the irradiation can be repeated a number of times. The repeating number or the number of irradiation cycles can be adjusted to the structure development during the foregoing irradiation cycles. For example a structure is not yet well developed, than another cycle can be applied. An energy release per pulse can be about 5-12 microjoules, and a spot size can be about 5 to 40 micrometer in diameter. An advantage of this technique is that different areas or surfaces of a substrate can be provided with different dedicated surface structures, which is extremely difficult with etching or shot blasting.

In a preferred embodiment, the spot is moved over the surface in a velocity of 10 to 100 mm per second. With such a speed the generation of the protrusions on the surface proves most advantageous.

The wave length of the laser can be between 200 to 1100 nm, wherein promising results can for instance be obtained with a wavelength within 200 to 400 nm, such as for instance a wavelength of 355 nm. These wavelengths provide sufficient energy in order to shape the surface in question.

In a further preferred embodiment, the substrate comprises at least one component chosen from a group of a metal, a metalloid, silicon, a ceramic, a metallic compound, a metalloid compound, carbon, a carbon containing compound or an alloy. Herein, the ceramic can for instance comprise a component chosen from a group of a silicon carbide, a silicon nitride, a metal carbide, a metal nitride, a metalloid nitride, a metalloid carbide, carbon, a metal oxide or a metalloid oxide. These compounds are relatively inert and as such difficult to provide with surface treatments. Due to this inert nature, these compounds can be applied in in-vivo applications such as implants.

In yet another preferred embodiment is the irradiation spot moved over the surface of the substrate in a pattern, such that the structure of the surface varies along the surface. By this pattern a dedicated structure can be provided on selected areas, whereas other areas can be provided with different structures or when applicable with none.

The surface structure preferably comprises an open submicron structure having randomly placed protrusions of 0.1 to 10 micrometer in diameter and a roughness obtained by these protrusions of plus and minus 0.5 micron to plus minus 15 micrometer, in a direction perpendicular to the surface.

In a preferred embodiment at least a portion of the protrusions of the surface structure comprises an overhanging part, which provides superior bonding properties. These overhanging parts can provide engaging spaces which can provide superior bonding properties.

Preferably the density and dimensions of the protrusions vary along the surface. With this variation, several structures can be provided with different properties. In another preferred embodiment a bone implant is provided with the above described surface structure.

The invention will now be elucidated by the following figures wherein:
Figure 1 represents a sectional view of a surface structure according to the state of the art;
Figures 2-4 represent different magnifications of an electron microscopic picture in top view of a surface structure as currently applied in the state of the art;
Figure 5 represents a schematic three dimensional view of the method according to the invention;
Figure 6 represents a surface structure obtained according to a first embodiment of the invention;
Figures 7 to 9 represent different magnifications of an electron microscopic picture in top view of a surface structure obtained according to a further embodiment of the invention ;
Figures 10 to 13 represent different magnifications of an electron microscopic picture in top view of a surface structure obtained according to a further embodiment of the invention;
Figures 14 to 17 represent different magnifications of an electron microscopic picture in top view of a surface structure obtained according to yet another embodiment of the invention;

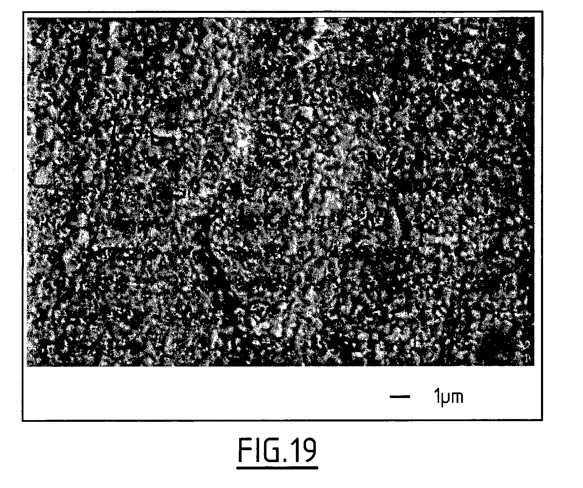

Figures 18 and 19 represent different magnifications of an electron microscopic picture in top view of an untreated surface.

The figures represent detailed embodiments and should not be considered limiting the invention in any way or form. Throughout the figures similar or the same reference signs are used for corresponding features.

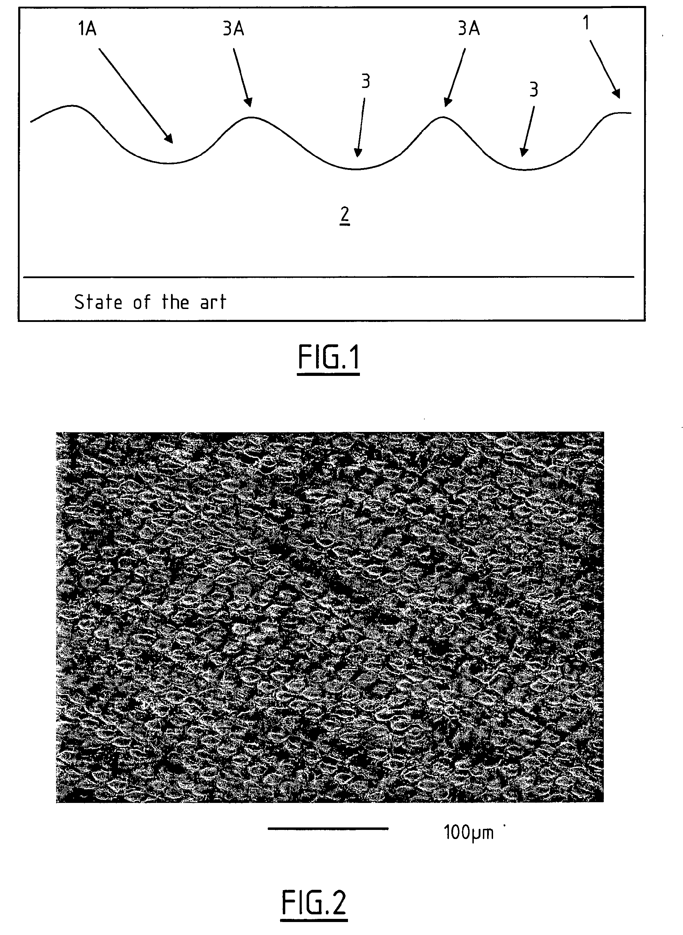

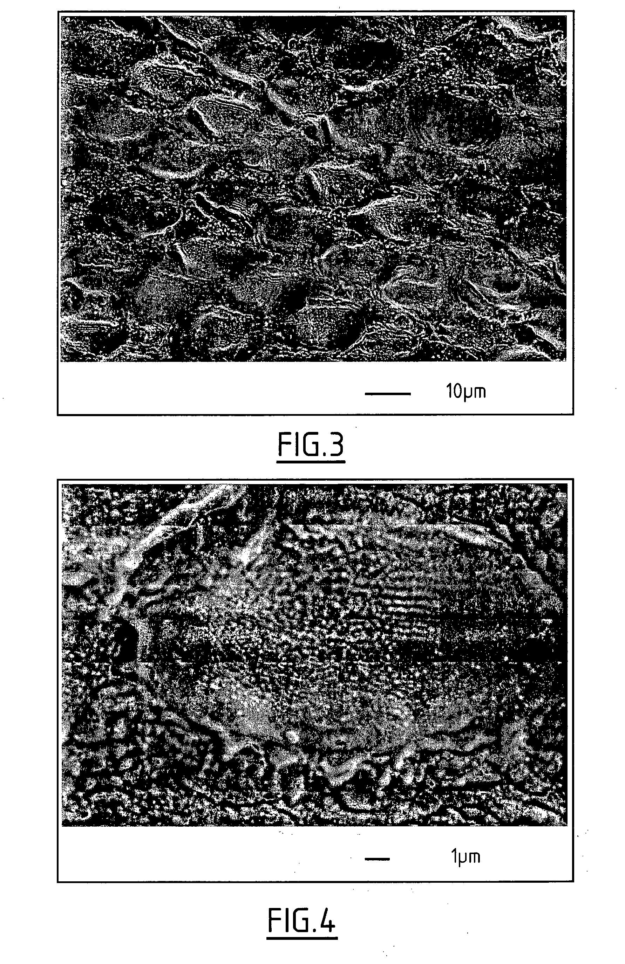

In figure 1 and the electron microscopic pictures represented by figures 2-4, an object 2 with a surface structure 1a is depicted that is currently applied as for instance a bone implant such as e.g. a dental implant for mounting a replacement tooth. Such a surface structure 1a is generated by irradiating the surface 1 with a nanosecond pulsed UV laser. On the surface 1, a structure of dimples or craters 3 is formed. The size of dimples or craters 3 typically corresponds with the size of the spot 14 of the employed laser beam 13, being between 5 and 40 micrometer in diameter.

These craters or dimples 3 are surrounded by walls or embankments 3a, which form a pattern on the surface 1. Since a laser 12 is applied in order to generate this surface structure 1a, contamination of foreign substances or particles can be avoided.

However a thus generated surface structure 1a can still be relative easily loosened from the bone tissue growing thereon, since no overhang of the walls or the embankments 3a is present. Therefore no real engagement between the bone tissue and the surface structure 1a is possible.

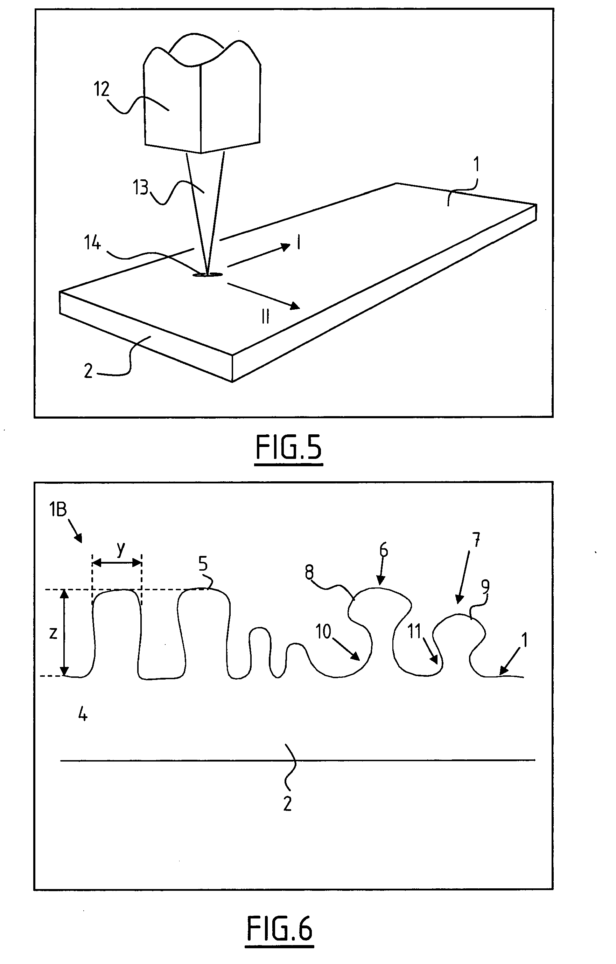

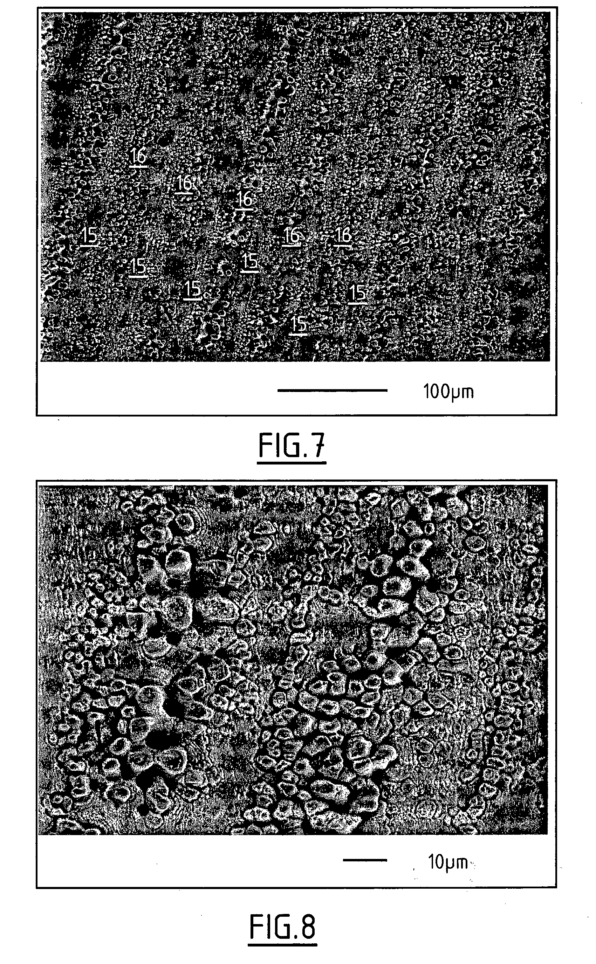

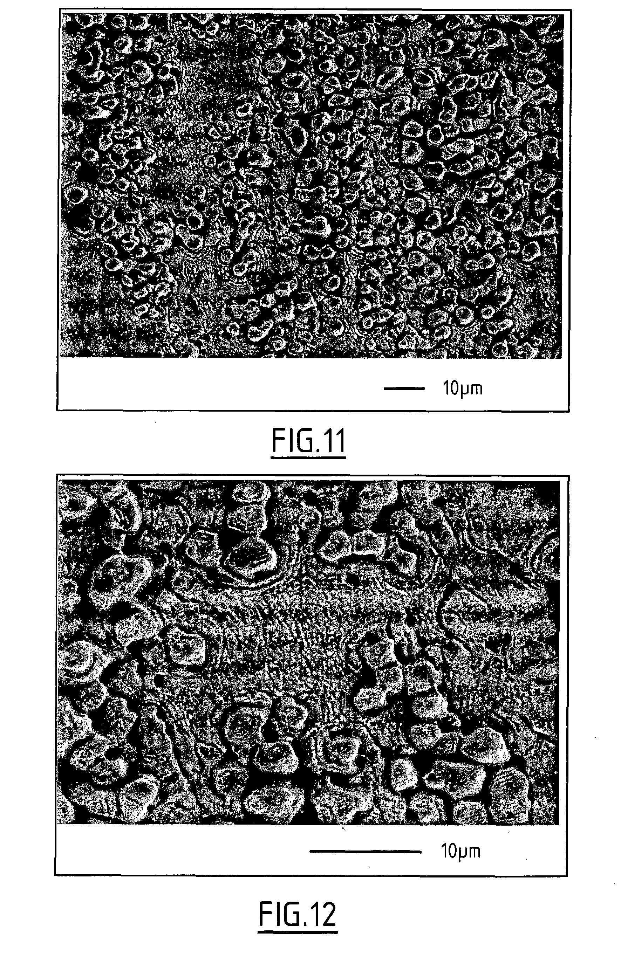

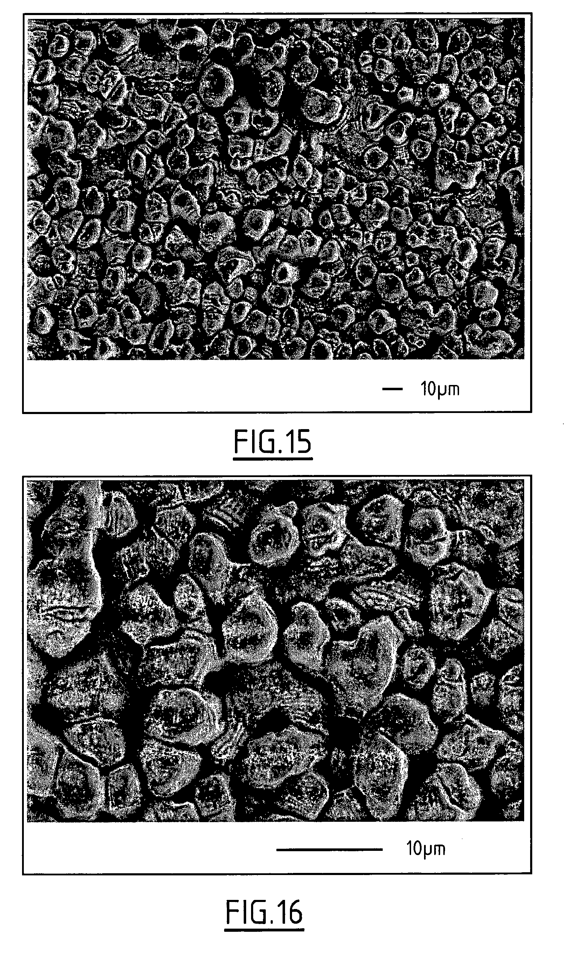

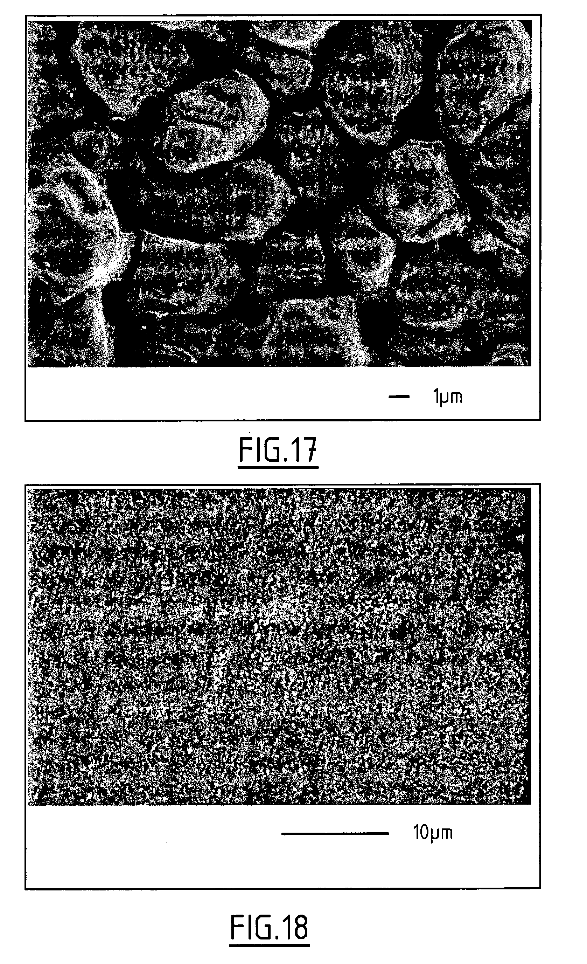

Surprisingly, by applying a laser 12 with a pulse length in the picoseconds range, different structures 1b can be made, which are represented in figure 6 as a sectional side view and in figures 7-17 as top view images of electron microscopic pictures according to different embodiments of the surface structures 1b.

These surface structures 1b are provided with microscopic pillars and protrusions 4,5,6,7 in the micrometer range. The dimensions of the protrusions 4,5,6,7 are found to be only a fraction of the spot size of the laser employed.

When figure 2, representing a surface structure 1a according to the state of the art is compared with any of surface structures 1b of the figures 7, 10 or 14, in the very same magnification, a more fine structure 1b can be observed. The protrusions 4,5,6,7 are in dimensions seen parallel to the surface less than 1 to several micrometers, whereas the craters or the dimples 3 in the state of the art are 10 micrometer or more.

So a much finer surface structure 1b is obtained, wherein, at least a portion of the protrusions can be provided with overhanging heads 8,9 so as to build mushroom or papilla shaped micro pillars 6,7. Thus necks 10 and 11 provide wider openings into which the bone tissue can grow and securely engage.

Without being bound to any theory, by applying a laser having a pulse frequency in the picosecond range, it is believed that a region on the surface 1, covered by the spot 14 of irradiation of the laser 12 in question, melts, resonates and breaks up such that the micro protrusions 4-8 are formed.

The height z of the protrusions 4-8 lies within a range of 1-15 micrometer, whereas the diameter y of the protrusions 4-8 is in the range of 0.1 to 10 micrometer. Thus a calculated surface roughness Ra in the range of 0 to 10 µm can be obtained.

By moving the laser spot 14 over the surface 1 in a certain pattern, a pattern of varying density and sizes of the protrusions 4,5,6 and 7 can be generated as can be seen in the lowest magnification electron microscopic pictures of the figures 7, 10 and 14.

In figure 7 for instance a pattern of repeating rows 15 of high density protrusions 4-7 are separated by areas 16 of lower density and smaller protrusions. The spot 14 of the picosecond pulsed laser 13 can be moved in a first direction I over the areas covering the rows 15, whereas the areas 16 received no or at least less irradiation. This way a pattern is obtained of which the calculated roughness is directionally oriented. In the first direction I (see figure 5), for instance a R_{A} value of 0.8 can be obtained, whereas in a second direction II (see figure 5) for instance a R_{A} value of 1.3 can be obtained.

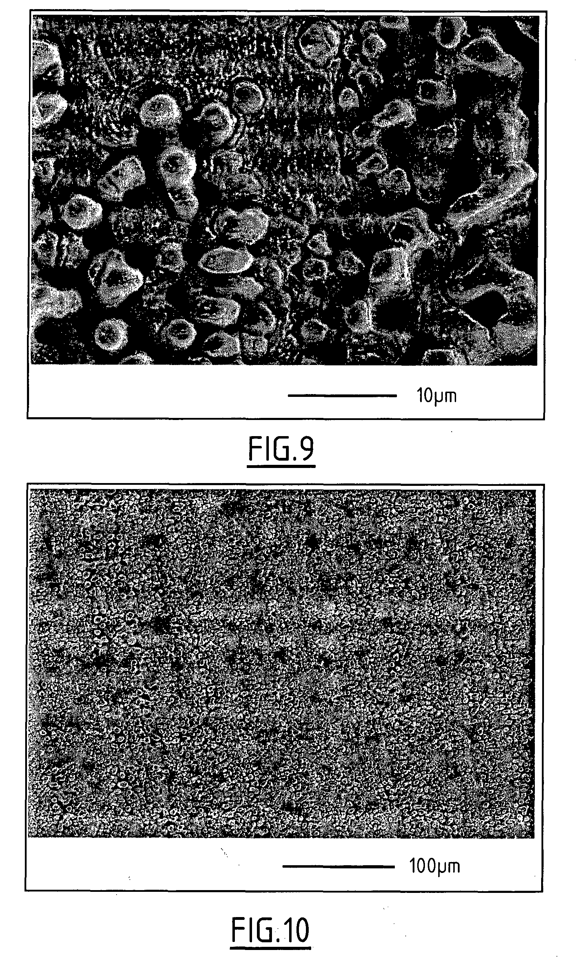

In figure 10, the rows 16 of high density protrusions 4-7 are put closer together by leaving less space for the areas that are not irradiated. Thus a more uniform, still slightly varying pattern is obtained. In this case the spot 14 of the laser 12 is moved in lanes in direction I, while the distance in between the lanes in direction II is reduced.

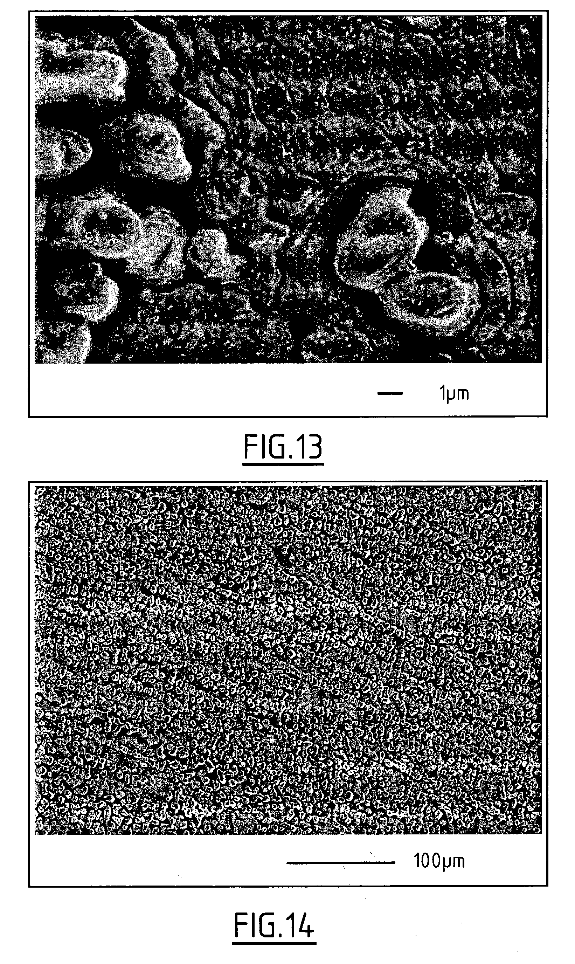

In figure 14, a linear pattern still remains visible whereas the density of the protrusions is almost uniform over the surface. In this case the distance 16 between the lanes 15 in the direction II is reduced to approximately zero.

Other patterns can similarly be made by applying corresponding paths of movement of the laser spot over the surface.

The invention is not to be considered restricted to the above described embodiments. Numerous adaptations and modifications are possible within the framework of the invention. For instance, multiple lasers of different pulsing frequency, wave length and/or other parameters could be applied on different areas of the surface or consecutive steps on the same or overlapping areas of the surface to be treated. Although the surfaces as described herein are disclosed to bone implants, the very same structures can be used in other surface finishing techniques, where a layer of protecting agent such as paint is applied to a surface, or when parts are glued together, in order to greatly enhance the adhesion of the glue to the surface in question.

For instance, in aeronautics, fuselage and/or wing parts can be glued together more sturdily and durable when the herein described surface structures are applied.

Also in ship building, for instance hulls can be glued together, wherein the hull portions that are glued together can be treated by a method as described above. Furthermore, in order to increase the efficiency of solar photovoltaic cells, a surface structure can be advantageous in order to increase the surface capture of photons. Alternatively a thus obtained surface can be used as drag reducing surfaces for applications where fluids flow past such surfaces, as typically in vehicles, sailing, flying and all kinds of sports.

## Claims

1. Method for producing a surface structure (1b) comprising the following steps:
- providing a substrate (2) having a surface (1) to be treated;
- irradiating a portion of the surface (1) with a beam (13) of a pulsed ultra violet laser (12) projecting a spot (14) on the surface (1), wherein the energy density per pulse ranges from 0.003 to 0.5 microjoules per square micrometer;
- moving the spot (14) over the surface (1) to be treated;
- repeating the irradiation a number of times, **characterized in that** the pulsed UV laser (12) is having a pulse length of 5 to 10 picoseconds, and a pulse frequency of 200 to 300 kiloherz.

2. Method according to claim 1, wherein the spot (14) is moved over the surface (1) in a velocity of 10 to 100 mm per second.

3. Method according to any of the preceding claims, wherein the wave length of the laser (12) is 200 to 1100 nanometer.

4. Method according to any of the preceding claims, wherein the substrate (2) comprises at least one component chosen from a group of a metal, a metalloid, silicon, a ceramic, a metallic compound, a metalloid compound, carbon, a carbon containing compound or an alloy.

5. Method according to claim 4, wherein the ceramic comprises a component chosen from a group of a silicon carbide, a silicon nitride, a metal carbide, a metal nitride, a metalloid nitride, a metalloid carbide, carbon, a metal oxide or a metalloid oxide.

6. Method according to any of the preceding claims, wherein the irradiation spot (14) is moved over the surface (1) of the substrate (2) in a pattern, such that the structure (1b) of the surface (1) varies along the surface (1) .

## Patentansprüche

1. Verfahren zum Erzeugen einer Oberflächenstruktur (1b), aufweisend die folgenden Schritte:
- Bereitstellen eines Substrats (2), das eine zu behandelnde Oberfläche (1) aufweist,
- Bestrahlen eines Teils der Oberfläche (1) mit einem Strahl (13) eines gepulsten UV-Lasers (12), der einen Punkt (14) auf die Oberfläche (1) projiziert, wobei die Energiedichte pro Impuls von 0,003 bis 0,5 Mikrojoule pro Quadratmikrometer reicht,
- Bewegen des Punktes (14) über der zu behandelnden Oberfläche (1),
- Wiederholen der Bestrahlung eine Anzahl von
Malen,
**dadurch gekennzeichnet, dass** der gepulste UV-Laser (12) eine Impulslänge von 5 bis 10 Pikosekunden und eine Impulsfrequenz von 200 bis 300 Kilohertz hat.

2. Verfahren gemäß Anspruch 1, wobei der Punkt (14) mit einer Geschwindigkeit von 10 bis 100 mm pro Sekunde über der Oberfläche (1) bewegt wird.

3. Verfahren gemäß irgendeinem der vorhergehenden Ansprüche, wobei die Wellenlänge des Lasers (12) 200 bis 1100 Nanometer beträgt.

4. Verfahren gemäß irgendeinem der vorhergehenden Ansprüche, wobei das Substrat (2) mindestens eine Komponente aufweist, die aus einer Gruppe eines Metalls, eines Halbmetalls, Silizium, einer Keramik, einer Metallverbindung, einer Halbmetallverbindung, Kohlenstoff, einer kohlenstoffhaltigen Verbindung oder einer Legierung ausgewählt wird.

5. Verfahren gemäß Anspruch 4, wobei die Keramik eine Komponente aufweist, die aus einer Gruppe eines Siliziumkarbids, eines Siliziumnitrids, eines Metallkarbids, eines Metallnitrids, eines Halbmetallnitrids, eines Halbmetallkarbids, Kohlenstoff, eines Metalloxids oder eines Halbmetalloxids ausgewählt wird.

6. Verfahren gemäß irgendeinem der vorhergehenden Ansprüche, wobei der Bestrahlungspunkt (14) in einem Muster über der Oberfläche (1) des Substrats (2) bewegt wird, so dass die Struktur (1b) der Oberfläche (1) entlang der Oberfläche (1) variiert.

## Revendications

1. Procédé de fabrication d'une structure de surface (1b) comprenant les étapes suivantes consistant à :
- prévoir un substrat (2) comportant une surface (1) à traiter ;
- irradier une partie de la surface (1) avec un faisceau (13) d'un laser ultraviolet pulsé (12) projetant un point (14) sur la surface (1), dans lequel la densité d'énergie par impulsion est située dans la plage de 0,003 à 0,5 microjoule par micromètre carré ;
- déplacer le point (14) sur la surface (1) à traiter ;
- répéter l'irradiation un certain nombre de fois, **caractérisé en ce que** le laser UV pulsé (12) a une longueur d'impulsion de 5 à 10 picosecondes et une fréquence d'impulsion de 200 à 300 kilohertz.

2. Procédé selon la revendication 1, dans lequel le point (14) est déplacé sur la surface (1) à une vitesse de 10 à 100 mm par seconde.

3. Procédé selon l'une des revendications précédentes, dans lequel la longueur d'onde du laser (12) est de 200 à 1 100 nanomètres.

4. Procédé selon l'une des revendications précédentes, dans lequel le substrat (2) comprend au moins un composant choisi dans un groupe constitué d'un métal, d'un métalloïde, de silicium, d'une céramique, d'un composé métallique, d'un composé métalloïde, de carbone, d'un composé contenant du carbone ou d'un alliage.

5. Procédé selon la revendication 4, dans lequel la céramique comprend un composant choisi dans un groupe constitué d'un carbure de silicium, d'un nitrure de silicium, d'un carbure de métal, d'un nitrure de métal, d'un nitrure de métalloïde, d'un carbure de métalloïde, de carbone, d'un oxyde de métal ou d'un oxyde de métalloïde.

6. Procédé selon l'une des revendications précédentes, dans lequel le point d'irradiation (14) est déplacé sur la surface (1) du substrat (2) selon un motif, de manière à ce que la structure (1b) de la surface (1) varie le long de la surface (1).
